# EUROPÄISCHE PATENTSCHRIFT

(11) **EP 0 548 132 B1**
(45) Veröffentlichungstag und Bekanntmachung des Hinweises auf die Patenterteilung: **04.01.1995**
(21) Anmeldenummer: 91915870.9
(22) Anmeldetag: 05.09.1991
(51) Int. Cl.: C07D 317/36, C25D 11/26

(54) **VERFAHREN ZUR HERSTELLUNG VON HOCHREINEM PROPYLENCARBONAT UND ZUR GLEICHZEITIGEN HERSTELLUNG VON PASSIVIERTEN ELEKTRODEN**
PROCESS FOR MAKING HIGHLY PURE PROPYLENE CARBONATE AND THE SIMULTANEOUS PRODUCTION OF PASSIVATED ELECTRODES
PROCEDE DE PRODUCTION SIMULTANEE DE CARBONATE DE PROPYLENE TRES PUR ET D'ELECTRODES PASSIVEES

(30) Priorität: 10.09.1990 DE 4028708
(43) Veröffentlichungstag der Anmeldung: 30.06.1993
(73) Patentinhaber: HÜLS AKTIENGESELLSCHAFT, 45764 Marl (DE)
(72) Erfinder: WOLFGANG, Dittrich, D-8000 München 127 (DE); HILL, Herbert, D-8058 Erding (DE); KINZEL, Elke, D-8011 Zorneding (DE)
(74) Vertreter: Goldbach, Klara, Dr.
(86) Internationale Anmeldenummer: DE9100719
(87) Internationale Veröffentlichungsnummer: WO9204340

(56) Entgegenhaltungen:
- GB-A- 928 820
- Pure and Applied Chemistry, vol. 27, No. 1-2, 1971, (London, GB), T. Fujinaga et al.: "Propylene carbonate: purification and tests for purity", pages 275-279, see pages 275, 276 below; page 278 (cited in the application)

## Beschreibung

Die vorliegende Erfindung betrifft ein Verfahren zur Herstellung von hochreinem Propylencarbonat und zur gleichzeitigen Herstellung von passivierten Elektroden.

Propylencarbonat (4-Methyl-1,3-dioxolan-2-on) ist ein farbloses, aprotisches Lösungsmittel mit einer relativ hohen Dielektrizitätszahl. Es wird in großem Umfang als Lösungsmittel für verschiedene synthetische Polymere und als Reaktionsmedium eingesetzt. Hauptsächlich wird Propylencarbonat als nichtwäßriges Lösungsmittel für Elektrolyte für Hochenergiebatterien verwendet.

Als Dielektrikum ist Propylencarbonat allgemein zur Verwendung in elektrostatischen Vorrichtungen geeignet. In elektrostatischen Vorrichtungen können hohe Energie- und Leistungsdichten erreicht werden, weg hohe elektrische Felder bei kleinen Injektions- bzw. Verlustströmen innerhalb des verwendeten Dielektrikums aufrechterhalten werden. Die elektrische Leitfähigkeit von handelsüblichem Propylencarbonat, die üblicherweise bei 10⁻⁶ bis 10⁻⁷ S/cm liegt, ist jedoch zu hoch, um die Verlustströme wesentlich zu verringern, so daß es in ungereinigtem oder grobgereinigtem Zustand nicht verwendbar ist.

Handelsübliches Propylencarbonat enthält als Hauptverunreinigungen Propylenoxid, Kohlendioxid, 1,2- und 1,3-Propandiol(Propylenglykol), Allylalkohol, Ethylencarbonat, Natriumionen, Reaktionsprodukte von Tetramethylammoniumbromid, Wasser und andere nicht identifizierte Substanzen. Die Methoden zur Bestimmung dieser Verunreinigungen, beispielsweise GC, IC oder MS, sind sehr aufwendig.

Es sind verschiedene Verfahren zur Reinigung von Propylencarbonat bekannt. So beschreiben T. Fujinaga und K. Izutsu in "Pure and Applied Chem. 27", (1971), Seiten 275 bis 280 und in "Recommended Methods for Purification of Solvents and Tests for Impuritives", Ed. J.F. Coetzel, Pergamon Press (1982), Seiten 19 bis 24, eine fraktionierte Destillation unter verringertem Druck, an die sich ein Verdampfungsverfahren anschließen kann, bei dem der Gehalt an niedrig siedenden organischen Bestandteilen auf unterhalb 0,4 ppm und der Wassergehalt auf unterhalb 1,5 ppm verringert werden kann. Die Glykolkonzentration verändert sich dabei jedoch nicht. Bei diesen Verfahren wird ein Propylencarbonat mit einer Leitfähigkeit von etwa 10⁻⁷ bis 10⁻⁸ S/cm erhalten.

Vor der fraktionierten Destillation kann eine Trocknung mit Molekularsieben, aktiviertem Aluminiumoxid bzw. getrocknetem Calciumoxid erfolgen (Y. Mary, Revue de Chim. Minerale 13 (1978), Seite 185 und L.M. Mukherjee, CRC Critical Rec. in Anal. Chem. 77 (1971), S. 345).

Alternativ dazu kann eine chemische Vorreinigung mit Kaliumpermanganat durchgeführt werden, bei der MnO₂ ausfällt und das überschüssige KMnO₄ durch Erwärmen bei 120°C zerstört wird. Anschließend erfolgt eine Vakuumdestillation.

Elektrodialysemembranen können ebenfalls zur Reinigung von Propylencarbonat verwendet werden (Influence de la pureté du carbonate de propylène sur sa résistivité électrique, A. Denat, B. Gosse und J.P. Gosse, Journal de chimie physique, 1975, 72, Nr. 3, S. 343 bis 346).

Elektrodialysemembranen weisen jedoch die folgenden Nachteile auf: Sie werden bei elektrischen Durchschlägen, die in elektrostatischen Vorrichtungen nicht auszuschließen sind, zerstört, d.h. sie kleben beispielsweise zusammen. In vielen Fällen weisen sie uneinheitliche Qualität auf, was zu Problemen beim Betrieb der elektostatischen Vorrichtungen führt. Es ist auch nur unter sehr hohem konstruktiven Aufwand möglich, unebene Elektrodenflächen mit Membranen zu bespannen. Elektrodialysemembranen sind im allgemeinen nicht für hohe Spannungen ausgelegt. Sie werden normalerweise nur im Voltbereich verwendet. Die Reinigung bzw. Entionisierung von Propylencarbonat ist nicht immer zufriedenstellend, da aufgrund der Lagerung der Membranen in wäßriger Lösung nach ihrem Trocknen bzw. Überführen in ein organisches Lösungsmittel ein erhöhter Wassergehalt in dem Propylencarbonat vorliegt.

Es ist bereits bekannt, Flüssigkeiten einem elektrischen Feld bei kleinen Feldstärken unter Verwendung von Elektroden auszusetzen. So beschreibt beispielsweise A. Nikuradse (Sonderabdruck aus Isolieröle, herausgegeben von Rhenania-Ossag Mineralölwerke AG; Hamburg, Julius Springer, Berlin, 1937) die Elektrizitätsleitung in Isolierölen. F. Wollers, "Injektionsstrom und Laufzeitmessung in flüssigem Benzol", Dissertation 1971, beschreibt den zeitlichen Verlauf des Stroms bei kleinen Feldstärken unmittelbar nach Anlegung einer Gleichspannung für Metallelektroden in flüssigem Benzol. Benzol ist jedoch unter anderem aufgrund seiner sehr niedrigen Dielektrizitätszahl als Dielektrikum in elektrostatischen Vcrrichtungen nicht geeignet.

Aufgabe der vorliegenden Erfindung ist es, ein zuverlässiges Verfahren zur Reinigung von Propylencarbonat zur Verfügung zu stellen, bei dem Propylencarbonat mit einer ausreichend verringerten Leitfähigkeit erhalten wird, so daß es zur Verwendung als Dielektrikum in elektrostatischen Vorrichtungen geeignet ist.

Diese Aufgabe wird durch ein Verfahren gemäß den Patentansprüchen gelöst.

Das erfindungsgemäße Verfahren führt überraschend zu einer drastischen Verringerung der Leitfähigkeit von Propylencarbonat unter gleichzeitiger Passivierung der Elektroden. Sowohl die erfindungsgemäß erhaltene Elektrode als auch das erfindungsgemäß erhaltene Propylencarbonat, das eine Leitfähigkeit im Bereich vom 10⁻¹⁰ bis 10⁻¹⁴ S/cm besitzt, eignen sich zur Verwendung innerhalb elektrostatischer Vorrichtungen.

Das Propylencarbonat mit geringer Leitfähigkeit führt dabei zu einer starken Reduzierung der Verlustströme. Der Vorteil der passivierten Elektroden bei Verwendung in elektrostatischen Vorrichtungen liegt in ihrer erhöhten Durchschlagsfestigkeit sowie ihrer verringerten Injektionsfähigkeit, die wiederum zu geringeren Verlustströmen führt. Insgesamt ergibt sich somit eine sofortige Einsatzbereitschaft der elektrostatischen Vorrichtung bei voller Leistung.

Es wird angenommen, daß durch das erfindungsgemäße Verfahren unter anderem eine ionische Dissoziation der Verunreinigungen des Propylencarbonats stattfindet, bei der sich die Ionen an den Elektroden entladen bzw. sich dort ablagern, d.h. es entsteht unter anderem ein Dissoziationsstrom. Dabei korreliert das Stromintegral bei ruhendem Flüssigkeitsvolumen mit den anfänglich vorhandenen ionischen Verunreinigungen im Propylencarbonat. Die Dissoziationsgeschwindigkeit steigt im allgemeinen mit dem elektrischen Feld, kann jedoch von Stoff zu Stoff unterschiedlich sein. Der Stromverlauf kann mittels eines Speicheroszillographen oder mittels eines mechanischen Schreibers registriert werden.

Gleichzeitig wird in dem erfindungsgemäßen Verfahren eine Passivierung der Elektroden erzielt, wobei die Reinigung des Propylencarbonats umso wirksamer erfolgt, je stärker die Passivierung der Elektroden fortschreitet.

Unter "blanken" Elektroden sind erfindungsgemäß handelsübliche, jungfräuliche Elektroden zu verstehen, die vor Gebrauch poliert, gereinigt und getrocknet werden. Sie werden üblicherweise mit einem Polierstein poliert, im Ultraschallbad mit Wasser und Aceton gereinigt und unter Vakuum getrocknet und weisen eine Rauhtiefe von weniger als 30 µm auf.

Unter "Passivierung" der Elektroden ist in der vorliegenden Erfindung eine Veränderung der Oberfläche der Elektroden zu verstehen, die chemischer und/oder physikalischer Art sein kann. Der Mechanismus dieser Passivierung ist noch nicht vollständig geklärt. Jedoch wird angenommen, daß sich bei dem erfindungsgemäßen Verfahren entweder eine Überzugsschicht auf den Elektroden ausbildet und/oder eine Oberflächenschicht abgetragen wird. Entscheidendes Kriterium für eine vorliegende Passivierung ist eine wesentliche Verringerung der Injektionsströme bei Verwendung der passivierten Elektroden in einer elektrostatischen Vorrichtung.

Fig. 1 zeigt eine schematische Darstellung einer Kreislaufführung des erfindungsgemäßen Verfahrens in einer Reinigungszelle.

Fig. 2 ist ein Diagramm, das die Beziehung zwischen dem Strom bzw. Injektionsstrom und der Feldstärke in einer Reinigungszelle bei Verwendung von nach dem erfindungsgemäßen Verfahren hergestelltem Propylencarbonat und passivierten Elektroden im Vergleich zur Verwendung von mit Elektrodialysemembranen gereinigtem Propylencarbonat und nicht passivierten Elektroden zeigt.

Fig. 3 und 4 sind Diagramme, die die Abnahme der leitfähigkeit von Propylencarbonat mit der Zeit bei Anlegung einer elektrischen Feldstärke von 20 kV/cm zeigen, wobei in Fig.4 die Feldstärke zeitweise variiert wurde.

Gemäß einer ersten Ausführungsform kann das erfindungsgemäße Verfahren in einer einfachen Reinigungszelle, aus der das gereinigte Propylencarbonat dann zur Lagerung entnommen wird, durchgeführt werden.

Es ist jedoch gemäß einer zweiten Ausfühungsform bevorzugt, das so erhaltene Propylencarbonat in einen Teil einer elektrostatischen Vorrichtung zu überführen, in dem das erfindungsgemäße Verfahren unter weiterer Reinigung fortgesetzt wird, oder das erfindungsgemäße Verfahren direkt in einer elektrostatischen Vorrichtung durchzuführen.

Nachstehend werden die beiden erfindungsgemäßen Ausführungsformen näher beschrieben.

Bei Verwendung einer Reinigungszelle ist es bevorzugt, daß das Propylencarbonat im Kreislauf durch die Zelle strömt. Dies geschieht üblicherweise bei Raumtemperatur und Normaldruck. Eine inerte Atmosphäre, insbesondere eine Stickstoffatmosphäre, ist bevorzugt.

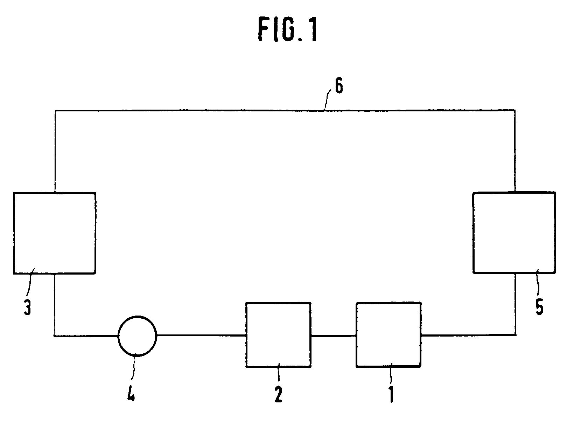

Bei der praktischen Durchführung des erfindungsgemäßen Verfahrens wird vorzugsweise zunächst die Dissoziationsgeschwindigkeit der ionischen Verbindungen des Propylencarbonats in Abhängigkeit von der elektrischen Feldstärke bestimmt. Wie in Fig. 1 gezeigt, wird in einer Testzelle 1 über einem ruhenden Flüssigkeitsvolumen von Propylencarbonat ein elektrisches Feld angelegt, wodurch unter anderem ein Dissoziationsstrom entsteht. Die Zeit, die benötigt wird, bis der Dissoziationsstrom annähernd Null ist, ist die minimale Reinigungszeit, die zum Reinigen des Propylencarbonats in der Reinigungszelle 2 erforderlich ist. Das vorgereinigte Propylencarbonat wird nun aus einem Vorratsbehälter 3 über eine Pumpe 4 in die Reinigungszelle 2 geleitet, in der es nach dem erfindungsgemäßen Verfahren behandelt wird, und weiter in einen Aufnahmebehälter 5.

Anstelle des Aufnahmebehälters 5 kann es direkt in eine elektrostatische Vorrichtung geleitet werden. Die Behälter 3 und 5 sind mit einer Ausgleichsleitung 6 verbunden, um ein mehrmaliges Durchlaufen der Reinigungszelle 2 zu gestatten, bzw. um das Propylencarbonat bereits während der Reinigung im Kreislauf durch die Reinigungszelle zu führen. Durch die dadurch erzielte Relativgeschwindigkeit zwischen durchströmendem Propylencarbonat und den feststehenden Elektroden können unerwünschte Ablagerungen an den Elektroden verhindert werden. Dies kann auch dadurch erreicht werden, daß mindestens eine Elektrode in Bewegung gehalten wird. Mittels der nach der Reinigungszelle 2 angeordneten Testzelle 1 kann laufend die Reinigungsgüte des Propylencarbonats geprüft werden. Die Atmosphäre über den Behältern besteht aus hochreinem inertem Gas, z.B. Stickstoff oder Edelgasen.

Vorzugsweise wird das Verfahren mit einem Gleichspannungsfeld durchgeführt.

Als Elektroden können handelsübliche Metall-, metallbeschichtete Kunststoff- oder metallbeschichtete Keramikelektroden verwendet werden. Beispiele für Metallelektroden sind Messing- und Platinelektroden.

Das elektrische Feld kann zeitlich bezüglich seiner Höhe gesteuert werden. Dabei ist ein gelegentlicher, nichtperiodischer Polaritätswechsel der angelegten Spannung, d.h. eine Umpolung des Feldes, bevorzugt. Üblicherweise werden Feldstärken von mindestens 1 kV/cm, bevorzugt 10 bis 100 kV/cm, besonders bevorzugt 15 bis 20 kV/cm, angelegt.

Die Stromdichte des sich während der Reinigung einstellenden Stroms kann nach oben begrenzt werden, vorzugsweise auf Werte von kleiner als 30 µA/cm².

Die in der Reinigungszelle verwendeten Elektroden besitzen vorzugsweise eine pilzförmige Form, d.h. sie weisen abgerundete Kanten auf. Die Elektrodenfläche beträgt beispielsweise 1 bis 5000 cm², insbesondere 10 bis 100 cm², und der Elektrodenabstand liegt vorzugsweise bei 0,5 mm bis 6 cm, insbesondere bei 1 mm bis 1 cm, besonders bevorzugt bei 4 bis 6 mm.

Der Durchsatz an Dielektrikum beträgt üblicherweise 0,4 bis 1,5 l/min.

Wird das erfindungsgemäße Verfahren gemäß der zweiten Ausführungsform, gegebenenfalls nach einer bereits erfolgten Reinigung in der Reinigungszelle, in einer elektrostatischen Vorrichtung, wie einem Impulsgenerator, ausgerüstet mit Stator- und Rotorelektroden, oder einem Kondensator, durchgeführt, so kann es unter den gleichen Bedingungen, wie vorstehend für die Reinigungszelle beschrieben, durchgeführt werden, wobei jedoch folgende Änderungen zweckmäßig sind.

Das erfindungsgemäße Verfahren wird im Impulsgenerator bevorzugt bei Temperaturen von 0 bis 10°C und einem Druck von 400 bis 600 kPa durchgeführt. Als Metallelektroden werden vorzugsweise Titan- oder Messingelektroden in Schalenform, die beispielsweise eine Elektrodenfläche von 19 bis 250 cm² aufweisen, verwendet. Eine bevorzugte Umdrehungszahl des Rotors liegt zwischen 1000 und 7500 Upmin. Der kleinste Abstand zwischen den Elektroden liegt bei 0,5 bis 2 mm, vorzugsweise bei 1 mm. Die elektrische Feldstärke beträgt mindestens 50 kV/cm, vorzugsweise 100 bis 330 kV/cm, besonders bevorzugt 200 bis 310 kV/cm.

Bei höheren Feldstärken, wie sie üblicherweise im Impulsgenerator verwendet werden, erfolgt die Reinigung des Propylencarbonats schneller als bei niedrigeren Feldstärken, wie etwa in der Reinigungszelle. So werden beispielsweise im Impulsgenerator 1 l Propylencarbonat bei 150 kV/cm in 10 bis 15 min gereinigt, während in der Reinigungszelle bei 20 kV/cm 12 h erforderlich sind, um jeweils ein Propylencarbonat mit einer Leitfähigkeit von 5 x 10⁻¹¹ S/cm zu erhalten.

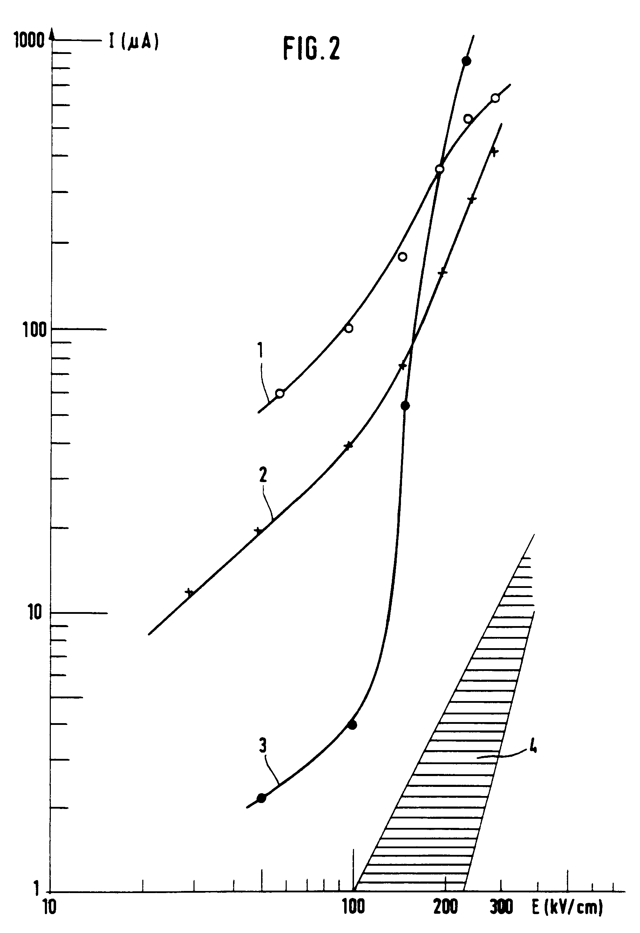

Wie Fig. 2 zeigt, werden Ströme in einer Zelle aufgrund der besonders guten Passivierung der Elektroden bei Verwendung von Propylencarbonat mit einer Leitfähigkeit von 1 x 10⁻¹¹ bis 2 x 10⁻¹¹ S/cm wesentlich reduziert. Die angelegte Spannung betrug 20 kV, und der Elektrodenabstand war 1 mm.

Die Kurven 1 bis 3 zeigen die sich bei bestimmten Feldstärken einstellenden Ströme, erhalten für mit Elektrodialysemembranen vorgereinigtes Propylencarbonat einer Leitfähigkeit von 9 x 10⁻¹¹ S/cm (Kurve 1), 6 x 10⁻¹¹ S/cm (Kurve 2) und 5 x 10⁻¹² S/cm (Kurve 3) und blanke Elektroden. Bereich 4 zeigt demgegenüber gemessene Ströme, wie sie sich bei Propylencarbonat mit einer Leitfähigkeit von 1 x 10⁻¹¹ bis 2 x 10⁻¹¹ S/cm und passivierten Elektroden einstellen.

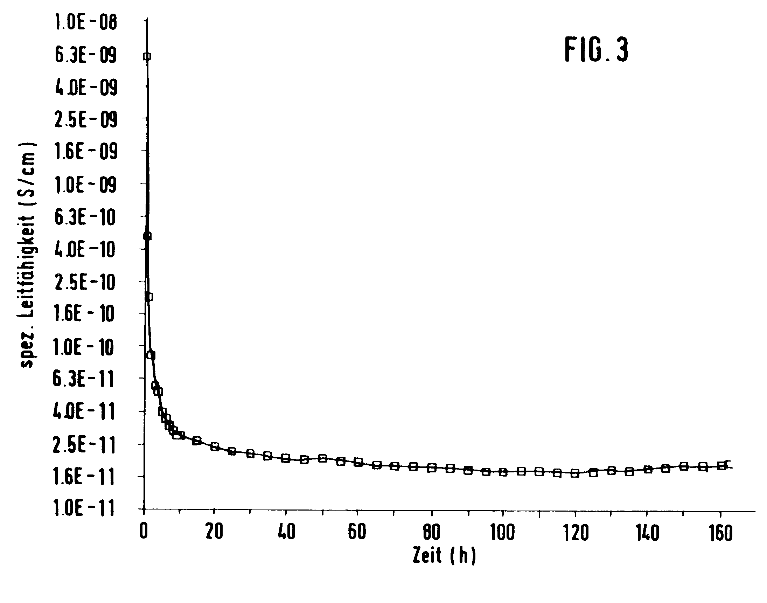

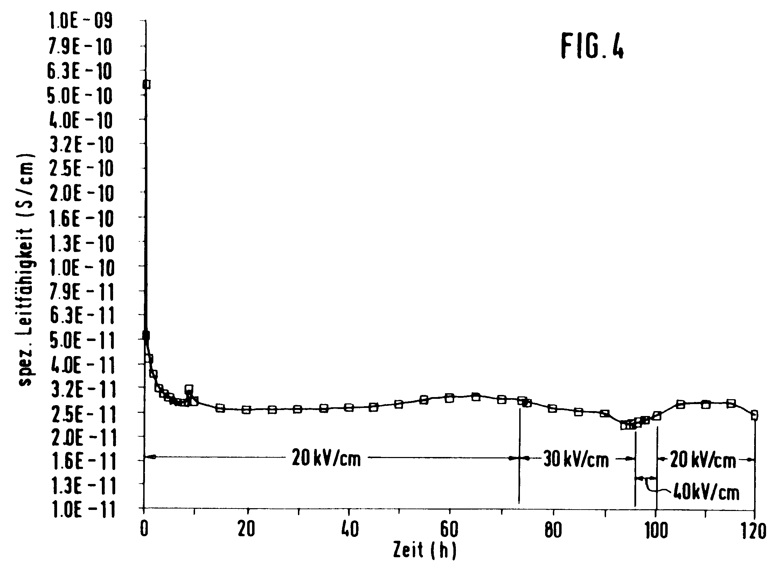

Fig. 3 und 4 zeigen die Verringerung der Leitfähigkeitzwerte des Propylencarbonats in dem erfindungsgemäßen Verfahren. Die Anfangsleitfähigkeit des vorgereinigten Propylencarbonats gem. Fig. 3 betrug etwa 6 x 10⁻⁹ S/cm und konnte in dem erfindungsgemäßen Verfahren durch Anlegen einer Spannung von 10 kV unter Verwendung von blanken Messingelektroden mit 0,5 cm Abstand nach etwa 10 h auf etwa 2,8 x 10⁻¹¹ S/cm verringert werden. Im Kreislauf befanden sich 2 l Propylencarbonat. 90 h nach Abschalten der Spannung stellte sich ein Leitfähigkeitswert von etwa 10⁻¹⁰ S/cm ein. Die Leitfähigkeit konnte somit um das etwa 100fache vermindert werden.

Fig. 4 zeigt ein weiteres Beispiel der Reinigung von Propylencarbonat mit blanken Messingelektroden unter Variierung der Spannung (10, 15, 20, 10 kV), wobei eine Verringerung der Leitfähigkeit von etwa 5 x 10⁻¹⁰ auf etwa 3 x 10⁻¹¹ S/cm erhalten wurde.

Das verwendete Propylencarbonat kann auf bekannte Weise vorgereinigt werden, beispielsweise durch Umsetzung mit Kaliumpermanganat und anschließende Vakuumdestillation.

Die folgenden Beispiele erläutern die Erfindung.

### Beispiel 1

4 l handelsübliches Propylencarbonat mit einer Leitfähigkeit von etwa 2 x 10⁻⁷ S/cm werden mit einem Überschuß an Kaliumpermanganat (15 g/l Propylencarbonat) 8 h gerührt. Anschließend wird über einen Faltenfilter filtriert und je 2 l der noch violett gefärbten Lösung unter Stickstoffatmosphäre über eine mit Aluminiumoxid (neutral, Aktivität 1, 600 g/2 l Propylencarbonat) gefüllte Säule mit einem Durchmesser von 5 cm gereinigt. Dabei wird ein Propylencarbonat mit einer Leitfähigkeit von etwa 1 x 10⁻⁸ S/cm erhalten.

Anschließend wird das Propylencarbonat einer raschen Vakuumdestillation bei einem Druck von 100 Pa unterworfen. Die erste Fraktion von etwa 500 ml als Vorlauf wird verworfen, und die nächste Fraktion von etwa 2 l Propylencarbonat mit einer spezifischen Leitfähigkeit von etwa 5 x 10⁻¹⁰ S/cm wird mittels Hochspannung weiter gereinigt. Dazu wird das Propylencarbonat unter Stickstoffatmosphäre in einem Kreislauf 24 h durch eine Reinigungszelle mit blanken Messing-Elektroden gepumpt, die mit Hochspannung beaufschlagt wird. Die elektrische Feldstärke beträgt 20 kV/cm, der Elektrodenabstand 0,5 cm und die Elektrodenfläche 15 cm². Das erhaltene Propylencarbonat besitzt eine Leitfähigkeit von etwa 10⁻¹¹ S/cm.

### Beispiel 2

Messing-Elektroden mit einer Elektrodenfläche von 15 cm² werden poliert und im Ultralschallbad mit Wasser und mit Aceton gereinigt sowie im Vakuum getrocknet. Nach Einbau in eine Reinigungszelle wird der Elektrodenabstand auf 5 mm eingestellt und die Zelle in einen Kreislauf mit Pumpe und Propylencarbonat-Vorratskolben integriert.

500 ml wie in Beispiel 1 vorgereinigtes Propylencarbonat werden bei Raumtemperatur und Normaldruck unter Stickstoffatmosphäre mit einer Geschwindigkeit von 0,75 l/min durch die Zelle gepumpt. Die Zelle wird mit Hochspannung beaufschlagt, wobei die Spannung bei einem Maximalstrom von 100 µA bis zur Endspannung von 10 kV erhöht wird. Die elektrische Feldstärke beträgt 20 kV/cm. Das Propylencarbonat wird 12 h durch die Zelle gepumpt und dadurch die Elektroden passiviert. Das erhaltene Propylencarbonat besitzt eine Leitfähigkeit von 2 x 10⁻¹¹ S/cm.

### Beispiel 3

4 l handelsübliches Propylencarbonat mit einer Leitfähigkeit von etwa 2 x 10⁻⁷ S/cm werden mit einem Überschuß an Kaliumpermanganat (15 g/l Propylencarbonat) 8 h gerührt. Anschließend wird über einen Faltenfilter filtriert und je 2 l der noch violett gefärbten Lösung unter Stickstoffatmosphäre über eine mit Aluminiumoxid (neutral, Aktivität Super 1, 300 g/21 Propylencarbonat) gefüllte Säule mit einem Durchmesser von 5 cm gereinigt. Dabei wird ein Propylencarbonat mit einer Leitfähigkeit vom 4 x 10⁻⁹ S/cm erhalten.

Anschließend wird das Propylencarbonat einer raschen Vakuumdestillation bei einem Druck von 100 Pa unterworfen. Die erste Fraktion von etwa 500 ml als Vorlauf wird verworfen, und die nächste Fraktion von etwa 2 l Propylencarbonat mit einer spezifischen Leitfähigkeit von 5 x 10⁻¹⁰ S/cm wird anschließend 4 Tage in einer Reinigungszelle mit Umlauf mit zwei blanken Messingelektroden bei einer Feldstärke von 20 kV/cm gereinigt. Im Anschluß werden 1,5 l Propylencarbonat mit nachgereinigtem Stickstoff in einen Impulsgenerator mit Messingelektroden einer Oberfläche von 19 cm² eingefüllt.

In einem gekühlten Kreislauf wird das Propylencarbonat bei einem Druck von 550 kPa mit einem Durchsatz von 1 l/min bei einer Drehzahl von 2000 Upmin durch den Impulsgenerator gepumpt. Die Propylencarbonattemperatur beträgt bei Versuchsbeginn 5°C und bei Versuchsende 31°C. Die anliegende Feldstärke beträgt 100 kV/cm über 3 min, 150 kV/cm über 3 min, 200 kV/cm über 3 min und 220 kV/cm über 5 min.

Die Leitfähigkeit des Propylencarbonats vor der Reinigung im Impulsgenerator beträgt 6 x 10⁻¹¹ S/cm, und nach 14 min elektrischer Reinigung bei den vorher angegebenen Feldstärken beträgt sie 2,5 x 10⁻¹² S/cm.

Eine erneute Propylencarbonatfüllung derselben Güte ergibt bei gleicher Anfangsleitfähigkeit nach 2 min elektrischer Reinigung im Impulsgenerator bei 2000 Upmin und einer Feldstärke von 180 kV/cm eine Leitfähigkeit von 2,5 x 10⁻¹² S/cm.

### Beispiel 4

2 l handelsübliches Propylencarbonat mit einer Leitfähigkeit von etwa 2 x 10⁻⁷ S/cm werden mit einem Überschuß an Kaliumpermanganat (15 g/l) 8 h gerührt. Anschließend wird über einen Faltenfilter filtriert. Nun wird die noch noch violett gefärbte Lösung unter Stickstoffatmosphäre über eine mit Aluminiumoxid (neutral, Woelm, Aktivität Super 1, 300 g/2 l Propylencarbonat) gefüllte Säule mit einem Durchmesser von 3 cm gereinigt. Dabei wird ein Propylencarbonat mit einer Leitfähigkeit vom 2 x 10⁻⁸ S/cm erhalten.

Anschließend wird das Propylencarbonat einer raschen Vakuumdestillation unterworfen. Die erste Fraktion von ungefähr 500 ml als Vorlauf wird verworfen, und von der nächsten Fraktion werden 500 ml in einen Impulsgenerator unter Stickstoffatmosphäre eingefüllt und gereinigt.

Die Propylencarbonattemperatur beträgt bei Versuchsbeginn 0°C und bei Versuchsende 35°C, und der Druck liegt bei 500 kPa. Die Drehzahl des Impulsgenerators beträgt 1800 Upmin. Die anliegende Feldstärke beträgt 110 kV/cm über 4 min, 160 kV/cm über 3 min, 230 kV/cm über 3 min, 275 kV/cm über 3 min und 310 kV/cm über 5 min. Als Rotormaterial wird Titan und als Statormaterial Messing verwendet. Die Elektrodenfläche beträgt 19 cm².

Die Leitfähigkeit des Propylencarbonats vor der elektrischen Reinigung im Impulsgenerator beträgt 1,5 x 10⁻¹⁰ S/cm und nach 17 min elektrischer Reinigung bei den vorher angegebenen Feldstärken 4 x 10⁻¹² S/cm.

Eine erneute Propylencarbonatfüllung derselben Güte ergibt bei derselben Anfangsleitfähigkeit nach 2,5 min elektrischer Reinigung im Impulsgenerator bei 2500 Upmin und einer Feldstärke von 250 kV/cm eine Leitfähigkeit von 3,6 x 10⁻¹² S/cm.

## Patentansprüche

1. Verfahren zur Herstellung von hochreinem Propylencarbonat und zur gleichzeitigen Herstellung von passivierten Elektroden, bei dem man, gegebenenfalls vorgereinigtes, Propylencarbonat durch eine Zelle mit blanken Elektroden, an die ein elektrisches Feld angelegt worden ist, strömen läßt.

2. Verfahren nach Anspruch 1, **dadurch gekennzeichnet,** daß man das Propylencarbonat im Kreislauf durch die Zelle strömen läßt.

3. Verfahren nach Anspruch 1 oder 2, **dadurch gekennzeichnet,** man mindestens eine Elektrode in Bewegung hält.

4. Verfahren nach einem der Ansprüche 1 bis 3, **dadurch gekennzeichnet,** daß man es mit einem Gleichspannungsfeld durchführt.

5. Verfahren nach einem der Ansprüche 1 bis 4, **dadurch gekennzeichnet,** daß man es bei Raumtemperatur und Normaldruck durchführt.

6. Verfahren nach einem der Ansprüche 1 bis 5 **dadurch gekennzeichnet,** daß man es unter inerter Atmosphäre durchführt.

7. Verfahren nach Anspruch 6, **dadurch gekennzeichnet,** daß man es unter Stickstoffatmosphäre durchführt.

8. Verfahren nach einem der Ansprüche 1 bis 7, **dadurch gekennzeichnet,** daß man Messing- oder Titanelektroden verwendet.

9. Verfahren nach einem der Ansprüche 1 bis 8, **dadurch gekennzeichnet,** daß man die Feldstärke zeitlich verändert.

10. Verfahren nach einem der Ansprüche 1 bis 9, **dadurch gekennzeichnet,** daß man die Stromdichte des sich während der Reinigung einstellenden Stroms nach oben begrenzt.

11. Verfahren nach Anspruch 10, **dadurch gekennzeichnet,** daß die Stromdichte kleiner als 30 µA/cm² ist.

12. Verfahren nach einem der Ansprüche 1 bis 11, **dadurch gekennzeichnet,** daß man schalen- oder pilzförmige Elektroden verwendet.

13. Verfahren nach einem der Ansprüche 1 bis 12, **dadurch gekennzeichnet,** daß man es bei einer elektrischen Feldstärke von mindestens 1 kV/cm durchführt.

14. Verfahren nach einem der Ansprüche 1 bis 13, **dadurch gekennzeichnet,** daß man Elektroden mit einem Elektrodenabstand von 0,5 mm bis 6 cm verwendet.

15. Verfahren nach einem der Ansprüche 1 bis 14, **dadurch gekennzeichnet,** daß man Elektroden mit einer Elektrodenfläche von 1 bis 5000 cm² verwendet.

16. Verfahren nach einem der Ansprüche 1 bis 12, **dadurch gekennzeichnet,** daß man als Zelle ein Teil einer elektrostatischen Vorrichtung verwendet, wobei das Verfahren gegebenenfalls während des Betriebs der elektrostatischen Vorrichtung durchgeführt wird.

17. Verfahren nach Anspruch 16, **dadurch gekennzeichnet,** daß man als elektrostatische Vorrichtung einen Impulsgenerator verwendet.

18. Verfahren nach Anspruch 16, **dadurch gekennzeichnet,** daß man als elektrostatische Vorrichtung einen Kondensator verwendet.

19. Verfahren nach Anspruch 17 oder 18, **dadurch gekennzeichnet,** daß man es bei einer elektrischen Feldstärke von mindestens 50 kV/cm durchführt.

20. Verfahren nach einem der Ansprüche 16, 17 oder 19, **dadurch gekennzeichnet,** daß man Elektroden mit einem minimalen Elektrodenabstand von 0,5 bis 2 mm verwendet.

21. Verfahren nach einem der Ansprüche 16, 17, 19 oder 20, **dadurch gekennzeichnet,** daß man es unter Polaritätswechsel durchführt.

22. Verfahren nach einem der Ansprüche 17, 19, 20 oder 21, **dadurch gekennzeichnet,** daß man den Impulsgenerator mit einer Drehzahl von 1000 bis 7500 Upmin betreibt.

23. Verfahren nach einem der Ansprüche 1 bis 22, **dadurch gekennzeichnet,** daß man das Propylencarbonat durch Umsetzung mit Kaliumpermanganat und anschließende Vakuumdestillation vorreinigt.

## Claims

1. Process for making highly pure propylene carbonate and the simultaneous production of passivated electrodes which comprises possibly pre-purified propylene carbonate being made to flow through a cell containing metallically bright electrodes to which an electric field has been applied.

2. Process according to Claim 1, characterized in that the propylene carbonate is made to circulate through the cell.

3. Process according to Claim 1 or 2, characterized in that at least one electrode is kept in motion.

4. Process according to any one of Claims 1 to 3, characterized in that it is carried out with a direct-current field.

5. Process according to any one- of Claims 1 to 4, characterized in that it is carried out at room temperature and atmospheric pressure.

6. Method according to any one of Claims 1 to 5, characterized in that it is carried out under an inert atmosphere.

7. Process according to Claim 6, characterized in that it is carried out under a nitrogen atmosphere.

8. Process according to any one of Claims 1 to 7, characterized in that brass or titanium electrodes are used.

9. Process according to any one of Claims 1 to 8, characterized in that the field strength is varied with time.

10. Process according to any one of Claims 1 to 9, characterized in that an upper limit is imposed on the current density of the current established during the purification.

11. Process according to Claim 10, characterized in that the current density is smaller than 30 µA/cm².

12. Process according to any one of Claims 1 to 11, characterized in that shell- or mushroom-shaped electrodes are used.

13. Process according to any one of Claims 1 to 12, characterized in that it is carried out at an electric field strength of at least 1 kV/cm.

14. Process according to any one of Claims 1 to 13, characterized in that electrodes with an electrode gap of from 0.5 mm to 6 cm are used.

15. Process according to anyone of Claims 1 to 14, characterized in that electrodes having an electrode area of from 1 to 5000 cm² are used.

16. Process according to any one of Claims 1 to 12, characterized in that the cell used is a part of an electrostatic appliance, the process being carried out, if required, during operation of the electrostatic appliance.

17. Process according to Claim 16, characterized in that the electrostatic appliance used is a pulse generator.

18. Process according to Claim 16, characterized in that the electrostatic appliance used is a capacitor.

19. Process according to Claim 17 or 18, characterized in that it is carried out at an electric field strength of at least 50 kV/cm.

20. Process according to any one of Claims 16, 17 or 19, characterized in that electrodes with a minimum electrode gap of from 0.5 to 2 mm are used.

21. Process according to any one of Claims 16, 17, 19 or 20, characterized in that it is carried out with a change of polarity.

22. Process according to any one of Claims 17, 19, 20 or 21, characterized in that the pulse generator is operated at a rotational speed of from 1000 to 7500 rpm.

23. Process according to any one of Claims 1 to 22, characterized in that the propylene carbonate is prepurified by reaction with potassium permanganate, followed by distillation under reduced pressure.

## Revendications

1. Procédé de production simultanée de carbonate de propylène très pur et d'électrodes passivées par lequel on fait parcourir du carbonate de propylène préalablement nettoyé, si besoin est, à travers une cellule munie d'électrodes nues, auxquelles un champ électrique a été appliqué.

2. Procédé selon la revendication 1, caractérisé en ce qu'on fait parcourir le carbonate de propylène à travers la cellule dans le circuit.

3. Procédé selon la revendication 1 ou 2, caractérisé en ce qu'on maintient au moins une électrode en mouvement.

4. Procédé selon l'une quelconque des revendications 1 a 3, caractérisé en ce qu'on l'effectue en présence d'un champ de tension continue.

5. Procédé selon l'une quelconque des revendications 1 a 4, caractérisé en ce qu'on l'effectue a température ambiante et à pression normale.

6. Procédé selon l'une quelconque des revendications 1 à 5, caractérisé en ce qu'on l'effectue sous une atmosphère inerte.

7. Procédé selon la revendication 6, caractérisé en ce qu'on l'effectue sous une atmosphère d'azote.

8. Procédé selon l'une quelconque des revendications 1 à 7, caractérise en ce qu'on utilise des électrodes de titane ou de laiton.

9. Procédé selon l'une quelconque des revendications 1 a 8, caractérisé en ce qu'on change l'intensité du champ en fonction du temps.

10. Procédé selon l'une quelconque des revendications 1 à 9, caractérisé en ce qu'on limite vers le haut la densité du courant qui apparait pendant le nettoyage.

11. Procédé selon la revendication 10, caractérisé en ce que la densité de courant est inférieure à 30 µA/cm².

12. Procédé selon l'une quelconque des revendications 1 à 11, caractérisé en ce qu'on utilise des électrodes en forme de champignon ou en forme de plaque ou de coque.

13. Procédé selon l'une quelconque des revendications 1 à 12, caractérisé en ce qu'on l'effectue en présence d'une intensité de champ électrique d'au moins 1 kV/cm.

14. Procédé selon l'une quelconque des revendications 1 à 13, caractérisé en ce qu'on utilise des électrodes dont l'écartement est compris entre 0.5 mm et 6 cm.

15. Procédé selon l'une quelconque des revendications 1 à 14, caractérisé en ce qu'on utilise des électrodes dont la surface est comprise entre 1 et 5000 cm².

16. Procédé selon l'une quelconque des revendications 1 à 12, caractérisé en ce qu'on utilise, comme cellule, une partie d'un dispositif électrostatique, ce qui permet d'effectuer le procédé au besoin pendant le fonctionnement du dispositif électrostatique.

17. Procédé selon la revendication 16, caractérisé en ce qu'on utilise un générateur d'impulsions comme dispositif électrostatique.

18. Procédé selon la revendication 16, caractérisé en ce qu'on utilise un condensateur comme dispositif électrostatique.

19. Procède selon la revendication 17 ou 18, caractérisé en ce qu'on l'effectue en présence d'une intensité de champ électrique d'au moins 50 kV/cm.

20. Procédé selon l'une quelconque des revendications 16, 17 ou 19, caractérisé en ce qu'on utilise des électrodes dont l'écartement minimal est compris entre 0,5 et 2 mm.

21. Procédé selon l'une quelconque des revendications 16, 17, 19 ou 20, caractérisé en ce qu'on l'effectue en présence de changements de polarité.

22. Procédé selon l'une quelconque des revendications 17, 19, 20 ou 21, caractérisé en ce qu'on fait fonctionner le générateur d'impulsions à une vitesse angulaire de 1000 à 7500 rotations par minute.

23. Procédé selon l'une quelconque des revendications 1 à 22, caractérisé en ce qu'on nettoie préalablement le carbonate de propylène par une conversion avec du permanganate de potassium suivie d'une distillation sous vide.
